# EUROPEAN PATENT SPECIFICATION

(11) **EP 0 884 054 B1**
(45) Date of publication and mention of the grant of the patent: **09.04.2003**
(21) Application number: 98500119.7
(22) Date of filing: 12.05.1998
(51) Int. Cl.: A61K 38/55

(54) **Fixed-dose association of an angiotensin-converting enzyme inhibitor and of a calcium channel antagonist for the treatment of cardiovascular illnesses**
Kombination in feste Dosiermenge von Angiotensin-Converting-Enzyme-Hemmern mit Calcium-Kanal Antagonisten zur Behandlung von Herz-Kreislauferkrankungen
Association à dosage fixe comprenant un inhibiteur de l'enzyme de conversion de l'angiotensine et un antagoniste des canaux à calcium pour le traitement de maladies cardiovasculaires

(30) Priority: 13.05.1997 ES 9701017
(43) Date of publication of application: 16.12.1998
(73) Proprietor: VITA-INVEST, S.A., E-08970 Sant-Joan-Despi (ES)
(72) Inventor: Micheto Escude, Luis Manuel, 08980 Sant Feliu de Llobregat, Barcelona (ES); Delgadillo Duarte, Joaquin Antonio, 08301 Mataro, Barcelona (ES); Canovas, Pedro, 08006 Barcelona (ES)
(74) Representative: Ponti Sales, Adelaida

(56) References cited:
- EP-A- 0 331 803
- EP-A- 0 334 264
- EP-A- 0 545 194
- WO-A-95/08987
- US-A- 4 703 038
- US-A- 5 573 780
- SWEET, C. S. ET AL: "Enalapril in experimental hypertension and acute heart failure: comparison with calcium channel blockers" J. CARDIOVASC. PHARMACOL. ( 1986 ), 8(SUPPL. 1), S15-S19 , XP002140850
- STAESSEN J.A. ET AL: "Ambulatory monitoring uncorrected for placebo overestimates long-term antihypertensive action." HYPERTENSION, (1996) 27/3 I (414-420). , XP000921342
- BAUER, FRöMMING, FüHRER: "Lehrbuch der Pharmazeutischen Technologie", 1999, WISSENACHAFTLICHE VERLAGSGESELLSCHAFT MBH STUTTGART, STUTTGART

## Description

### Field of the invention

The present invention relates to a fixed-dose association of an angiotensin-converting enzyme inhibitor (ACE inhibitor), enalapril, and of a calcium channel inhibitor (CCI), nitrendipine, to a method for the preparation of a pharmaceutical composition which includes said fixed-dose association and to the use thereof in the treatment of illnesses of the cardiovascular system, in particular arterial hypertension.

The compositions of the present invention are presented in the form of solid single doses with fixed quantities of enalapril and nitrendipine, so that a single administration, once a day, achieves the effect for 24 hours following said dose. The pharmaceutical preparations of the present invention have a therapeutic effect in the treatment of hypertension and other illnesses of the cardiovascular system when the quantities of the active ingredients, enalapril and nitrendipine, are smaller than the usual therapeutic doses of each medicament administered alone. The preparations of the invention have lower dose-related adverse effects than those which arise in the administration of larger doses of each of the active ingredients separately in order to achieve the same therapeutic effect. The pharmaceutical compositions of this invention simplify the administration regime and are more acceptable to the patient.

### Background of the invention

International consensus exists regarding the initial treatment of slight-moderate hypertension (AHT). Health-dietary measures are an inescapable first step in tackling the hypertense patient. If suitable control of blood-pressure figures are not achieved with these measures, a pharmacological treatment must be started.

The pharmacological treatments recommended as first choice have varied over the course of recent years. Initially, these were the thiadizic diuretics, to which β-blockers were subsequently added, and this situation has now been extended to other pharmacological groups such as the ACE inhibitors, the CCAs Calcium channel antagonist and the α₁-blockers. All the aforesaid pharmacological groups are of similar efficacy in respect of control of blood-pressure figures and are recognized as first-choice alternatives.

Single-drug therapy remains at present compulsory practice as first step in the pharmacological treatment of AHT. Where an initial therapy fails, the various committees of experts consider several possibilities. A satisfactory response is generally held to be that which keeps arterial tension figures below 140/90 mmHg. If this objective has not been achieved after a period of time ranging between 1 to 3 months, various possibilities are considered which, although basically similar, present some differences according to the body which proposes them.

Where the initial therapy fails, the WHO recommends that the initial drug be replaced by another belonging to a different group. Where there had been a partial response, it is felt preferable to add a second drug from another group at low dosages, instead of increasing the dose of the first.

Despite the undoubted benefits in terms of morbimortality to which the treatment of AHT gives rise, the results are not always as encouraging as might have been expected and the patient remains exposed to a greater risk of suffering cardiovascular complications than do patients of normal arterial tension. One of the factors which contributes to this relative failure of antihypertension therapy is deficient or less than optimum control in many cases of the hypertense patient as a result of the adverse reactions associated with high doses of antihypertension medicaments and failures of compliance arising out of multiple therapy.

Furthermore, 35-50% of patients suffering from hypertension do not present a satisfactory response to the initial single-medicament treatment (Medical Research Council Working Party. Trial of treatment of mild hypertension: principal results. Br. Med. J., 1985; 291: 97-104 and Moser M. The fifth report on the Joint National Committee on detection, evaluation, and treatment of high blood pressure: a critique. Primary Cardiol. 1993; 16: 66-73). One of the causes of this high lack of response in the treatment of AHT is the coming into action of counter-regulatory mechanisms which partially limit the antihypertensive effect. When any of the arterial tension regulating systems is modified a compensatory response arises from the other factors intervening in said control. Each pharmacological group acts more specifically on one of these mechanisms, so that one of the primordial justifications for combined treatment is that of having simultaneous effect on more than one of the factors which give rise to arterial hypertension.

Fixed-dose combinations of antihypertension medicaments should thus fulfil a triple objective: an increase of efficacy due to simultaneous action on more than one of the mechanisms regulating blood pressure; improvement in tolerance, due to their being administered in doses lower than those for each of their components separately; and improved therapeutic compliance due to a lower number of ingestions being necessary.

Hence the interest in developing the association of an ACE inhibitor and a CCA by seeking a synergic action in the reduction of blood pressure, a reduction of the adverse effects inherent to the CCAs, and improved therapeutic compliance due to administration being in a single dose.

The mechanism by which both active ingredients could be potentiated is complex. The CCAs give rise to a balance of negative sodium which stimulates the renin-angiotensin system, which effect is held up by the ACE inhibitors. Moreover, the administration of ACE inhibitors produces an increase of vagal tone which would offset the sympathetic activation and tachycardia induced by the CCAs of the dihydropyridine group.

The choice of enalapril as ACE inhibitor in the association of the invention is due to the fact that it reduces blood pressure in all degrees of essential and renovascular hypertension. It is at least as effective as other ACE inhibitors and other antihypertension drugs from other pharmacological groups such as diuretics, β-blockers, CCAs and α₁-blockers. Its efficacy and safety have been demonstrated in numerous comparative clinical trials, and it has been available in many countries for several years. The dose range normally used is from 5 to 40 mg, once a day. The usual initial dose is 10 mg once a day for slight arterial hypertension and 20 mg once a day for other degrees of arterial hypertension. The usual maintenance dose is 20 mg once a day, which can be increased up to a maximum of 40 mg once a day, depending on the individual needs of each patient. In patients who do not usually respond to single-drug therapy, another drug from a different pharmacological group can be added in order to provide an additional response.

The ACE inhibitors also improve the survival rate of patients with congestive heart failure and can prevent or delay the development of left-ventricle dilation and heart failure in patients with symptomatic and asymptomatic left-ventricle dysfunction. The haemodynamic changes associated with both acute and long-term treatment with enalapril in patients with congestive heart failure include reduction of systemic vascular resistance (from 20% to 45%), reduction of mean arterial pressure (from 7% to 15%), reduction of pulmonary capillary pressure (from 25% to 50%) and increased heart rate (from 25% to 30%). Mortality and morbidity rates following long-term treatment (over one year) improve by approximately 15% in patients with slight and moderate heart failure (study by SOLVD N. Engl. J. Med. 1987; 316: 1429-1435) and by approximately 30% in patients with severe heart failure (CONSENSUS study, N. Engl. J. Med. 1991; 325: 293-302). The enalapril doses recommended for this purpose are 2.5 mg/day initially, increasing up to 10 to 20 mg/day depending upon the clinical response.

Diabetic nephropathy is a clinical syndrome characterized by persistent proteinuria, progressive reduction of the glomerular filtration rate and increased arterial pressure. Preceding these changes there is a silent period of variable duration during which the diabetic patient shows persistent microalbuminuria. The presence of microalbuminuria is important in that it has been shown to be a predictive factor for the clinical development of diabetic nephropathy. In patients of normal tension with non-insulin-dependent or hypertense diabetes mellitus controlled with nifedipine, the addition of 5 mg/day enalapril significantly reduces the microalbuminuria, by 40% to 50%, over a period of 48 months. In monitoring of up to 5 years, enalapril stabilizes the microalbuminuria in patients with normal blood pressure with non-insulin-dependent diabetes better than does the placebo.

The choice of nitrendipine as CCA in the association of the invention is due to its possessing predominantly peripheral vasodilator properties, which induces sustained reductions of systolic and diastolic blood pressure. It has been observed in various clinical trials that nitrendipine reduces blood pressure in patients with slight-moderate hypertension and that this effect is sustained following long-term administration. In comparative trials with diuretics, β-blockers and other CCAs nitrendipine has been observed to have similar efficacy in the control of slight-moderate arterial hypertension. Nitrendipine has been commercially available in many countries for several years. The usual initial dose in patients with slight-moderate arterial hypertension is 5 to 20 mg once a day. In function of the response the dose can be adjusted between 5 and 20 mg once or twice a day, whether in single-drug therapy or combined with a diuretic or a β-blocker.

Several associations of ACE inhibitors and CCAs have been studied: cilazapril and nitrendipine (Nakanishi and col., Curr. Ther. Res. 1992; 52: 514-523), captopril and nitrendipine (Gennari and col., Cardiovasc. Drugs. Ther. 1989; 3: 319-325), enalapril and felodipine (Morgan and col., Kidney International 1992; 41 (suppl. 36): S78-S81). On the basis of these and other studies carried out on associations between ACE inhibitors and CCAs it can be concluded that combined treatment is more effective and better tolerated than single-drug treatment with each of the drugs separately.

Also described are different associations of ACE inhibitors and CCAs, of the dihydropyridine type, for the treatment of arterial hypertension in several patients, for example EP 488059, EP 180785, EP 265685, WO 9607400, EP 272177.

US-A-4703038 describes a combination of dihydropyridines with angiotensin-converting enzymes inhibitors. ACE-inhibiting compounds which may be mentioned are N-(1-(S)-etoxycarbonyl-3-phenylpropyl)-L-alanyl-L-proline L-proline or the maleate salt thereof (generic: enalapril) and a dihydropyridine which may be mentioned is the nitrendipine. According to said document, the two substances are administered in a combination of 2.5-15 mg (enalapril) and 10-20 mg (nitrendipine) per day for the treatment of cardiovascular illnesses, in particular arterial hypertension in mammals including man. However, said document does not solve the problem of low stability of enalapril and bad dissolution rate of nitrendipine.

EP-A-0545194 refers to a stable formulation of enalapril salt, a process for the preparation thereof and the use thereof. The enalapril salt (formula I) is prepared in such a manner that a compound of formula II (see abstract) is suspended in demineralized water, a stoichiometric amount of the corresponding sodium compound is added thereto, formulating additives are added to this enalapril sodium salt of formula I prepared in situ, and then the whole is homogenized and formulated. The dissolution rate of enalapril obtained according to said document is of 100% at 10 minutes. However, said document does not solve the problem of low stability of enalapril and bad dissolution rate of nitrendipine in an association of both components.

Similarly to document EP-A-0545194, US-A-5573780 also refers to a stable pharmaceutical solid composition comprising enalapril as the sodium salt, which is made by the steps of:
i) mixing enalapril maleate with a carrier, and alkaline sodium compound, and water
ii) drying the wet mass, and;
iii) further processing the resultant dried mass into tablets.
However, said document does not solve the problem of low stability of enalapril and bad dissolution rate of nitrendipine in an association of both components.

On the other hand, the simplest way for increasing the low solubility of active substances is by micronisation, which reduces the particle size and increases the surface area of a particle through extensive grinding (BAUER, FRÖMMING, FÜHRER: "Lehrbuch der Pharmazeutischen Technologie", 1999, Wissenachaftliche Verlagsgesellschaft mbh Stuttgart, Stuttgart). However, as it is already stated in the prior art (WO 9508987), at strongly aerophilic active substances, such as 1,4-dihydropyridine derivatives that act as calcium channels antagonists (eg. nitrendipine), micronisation does not result in an increase of the dissolution rate.

Thus, WO 9508987 discloses a process for preparation of solid dispersions and deposits with dihydropyridine type calcium antagonists containing as the active substance one or more 1,4-dihydropyridine derivatives such as nitrendipine in order to solve the problem of the bad solubility of nitrendipine. The process disclosed comprise the following steps:
a) preparing a solution or suspension of one or more 1,4-dihydropiridines (nitrendipine), one or more water soluble mono, di, oligo or polysaccharides and preferably one or more surfactants in a hydrophilic solvent,
b) providing at least one carrier selected from mono, di, oligo and polysaccharides, the carrier being preferably mixed with one or more disintegrators,
c) evaporating the solvent by spraying the solution or suspension (a) onto the carrier (b) under a stream of heated air.

Alternatively, the process can be prepared by
i) preparing a solution or suspension of one or more 1,4-dihydropyridines (nitrendipine), one or more water soluble mono, di, oligo or polysaccharides in a hydrophilic solvent, the solution or suspension preferably including one or more surfactants and/or one or more disintegrators, and
ii) evaporating the solvent.

In said document it is stated that the micronized state cannot be used as a solution to the bad solubility of nitrendipine. The solution proposed by said document is preparing a solution or suspension of one or more 1,4-dihydropiridines (nitrendipine) and evaporating the solvent by spraying the solution in order to solve the bad solubility of nitrendipine.

It must nevertheless be taken into account that the first requirements to be considered in the development of a fixed-dose association is that both components are compatible from the pharmacokinetic and pharmacodynamic points of view.

### Description of the invention

Taking account of the aforesaid background, in this invention a fixed-dose association of an angiotensin-converting enzyme inhibitor (ACE inhibitor), enalapril, and of a calcium channel antagonist (CCA), nitrendipine, as defined in claim 1 was developed, these being drugs which, administered alone, have extensively demonstrated efficacy and safety in the treatment of arterial hypertension and other cardiovascular illnesses. Moreover, enalapril and nitrendipine belong to different pharmacological groups with antihypertensive effect, so that combined administration thereof permits simultaneous action on more than one of the blood pressure regulatory mechanisms.

The present invention also relates to a new pharmaceutical composition as defined in claim 7 and the method for preparation of same for oral administration as defined in claim 8 and for use in the treatment of arterial hypertension and other illnesses of the cardiovascular system as defined in claim 10. This pharmaceutical composition consists in a single-dose form which contains fixed quantities of enalapril and nitrendipine.

The molecule corresponding to the enalapril (Formula I) possesses three chiral centres and there can therefore exist in eight different enantiomeric forms. The enantiomer known by the name of enalapril and used in this invention is 1-(N-((S)-1-ethoxycarbonyl-3-fenilpropyl) -L-alanil)-L-prolina. Enalapril salts can be used, such as salts with organic and inorganic acids (maleate, hydrochlorate, etc.) and salts with bases (sodium, potassium, magnesium salts).

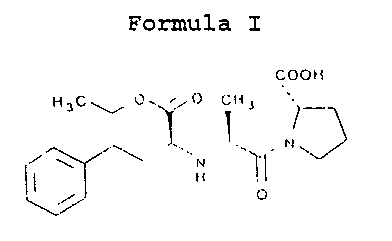

Nitrendipine (Formula II) has one chiral centre and, therefore, can be presented in two enantiometric forms. The commercial product is nevertheless the racemic mixture of the two isomers of ethyl and methyl ester of the acid 1,4-dihydro-2,6-dimethyl-4-(3-nitrophenyl)-3,5-pyridindicarboxylic. Nitrendipine salts can be used with organic and inorganic acids.

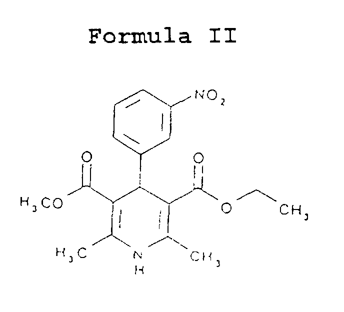

The instability of enalapril maleate and the considerable insolubility of nitrendipine are known. For this reason a method has been developed, and forms the object of this invention, for preparation of a gallenic formulation which achieves good stability of the enalapril, in the form of sodium salt, and good solubility of the nitrendipine, thereby achieving rapid release of the enalapril-nitrendipine association. The method for preparation of the formulation consists, on the one hand, in preparation of a granulating solution by dissolving the enalapril maleate and the sodium bicarbonate in a sufficient quantity of water. On the other hand, the remaining components are mixed with the exception of a fraction of the disintegrating excipients (starch, microcrystalline cellulose) and the lubricant (magnesium stearate). Said mixture is granulated with the aforesaid granulating solution. Following drying of the granulate a mass with a highly hydrophilic environment is obtained, which, linked with the action of the humectant (sodium lauryl-sulphate) favours dissolving of the nitrendipine. The agglutin and wetting agent (polyvinylpyrrolidone and sodium lauryl-sulphate) can as an option be incorporated into the granulating solution. A granulate is thus obtained which, following drying to a residual humidity of less than 1.5%, calibration and addition of the remaining excipients, can be compressed in a conventional press to provide stable tablets with rapid release of both active ingredients.

The dosage range of enalapril in the single fixed-dose forms of the association is between 2.5-20 mg, preferably between 10 and 20 mg. The dosage range of nitrendipine in the single fixed-dose forms fixed in the association is between 5-20 mg, preferably between 5 and 10 mg.

This form of single-dose administration facilitates the ingestion regime and improves acceptance by the patient.

Examples 1 and 2 below describe two different pharmaceutic formulations of the association of enalapril and nitrendipine object of the invention, together with the method for obtaining them. Example 3 describes the results of the trials on dissolution of the enalapril and nitrendipine of the formulations obtained in examples 1 and 2.

Example 4 describes the pharmacological effect of the association of enalapril and nitrendipine assessed in two experimental models: a) experimentally hypertense rat and b) beagle dog with normal blood pressure.

Example 5 describes the pharmacokinetic compatibility and example 6 the effectiveness of the pharmaceutical formulations of the invention in comparative clinical trials between enalapril, nitrendipine and a solid dosage form of the fixed-dose association of enalapril and nitrendipine.

### Description of figures

Figure 1 refers to the accumulative profile of in vitro dissolution of enalapril for the 6 tablets tested of the formulations of examples 1 and 2.

Figure 2 refers to the accumulative profile of in vitro dissolution of nitrendipine for the 6 tablets tested of the formulations of examples 1 and 2.

Figure 3 refers to the antihypertensive effect in rat hypertensive due to aortic coarctation. Arterial pressure (systolic and diastolic), mean values.

### EXAMPLE 1

This example describes the quantitative composition of a preferred formulation of the association of the invention, and the manufacturing method thereof.

| **Quantitative composition** | |
|---|---|
| ENALAPRIL MALEATE | 10.00 mg |
| MICRONIZED NITRENDIPINE | 10.00 mg |
| SODIUM BICARBONATE | 5.00 mg |
| CORN STARCH | 64.50 mg |
| SODIUM LAURYL-SULPHATE | 2.00 mg |
| MONOHYDRATED LACTOSE | 170.00 mg |
| POLYVINYLPYRROLIDONE | 8.00 mg |
| MICROCRYSTALLINE CELLULOSE | 33.00 mg |
| MAGNESIUM STEARATE | 1.15 mg |

### Manufacturing Method:

a) - Dissolve in a sufficient quantity of demineralized water the enalapril maleate and the corresponding quantity of sodium bicarbonate.
b)- Mix the nitrendipine suitably with 80 % of the corn starch, the sodium lauryl-sulphate, the monohydrated lactose, the polyvinylpyrrolidone and 20% of the monocrystalline cellulose, having previously sieved them.
c).- Using high-speed granulation equipment, granulate the products homogenized in section 2 with the solution obtained in section 1.
d).- Dry the granulated mass in fluidized bed equipment until a residual humidity of less than 1.5% is obtained.
e).- Calibrate the dry granulate. Add the remaining 20% of corn starch and the remaining 80% of microcrystalline cellulose and the magnesium stearate, previously sieved, and homogenize with the calibrated granulate.
f).- Compress in conventional press.

Tables 1 and 2 attached show the stability results of the enalapril and nitrendipine in this formulation in function of time. For this purpose, the formation of the products of degradation are quantified: dicetopiperazin and enalaprylic acid in the case of enalapril, and pyridinic derivative in the case of nitrendipine.

**Table 1.-**

| Stability results of the enalapril of the formulation of example 1 in function of time. | | | |
|---|---|---|---|
| | | Degradation products | |
| | Content in % of Enalapril | Dicetopiperazin | Enalaprylic acid |
| Initial amb. T. | 103.1 | 0.04 | 0.04 |
| 3 months amb. T. | 103.7 | 0.00 | 0.08 |
| 3 months 40°C | 102.3 | 0.04 | 0.12 |
| 3 months 40°C + 75% RH | 102.6 | 0.04 | 0.12 |
| 3 months 50°C | 102.3 | 0.19 | 0.41 |

**Table 2.-**

| Stability results of the nitrendipine of the formulation of example 1 in function of time. | | |
|---|---|---|
| | Content in % of Nitrendipine | Degradation product Pyridinic derivative |
| Initial amb. T. | 100.8 | 0.00 |
| 3 months amb.T. | 99.0 | 0.05 |
| 3 months 40°C | 102.2 | 0.02 |
| 3 months 40°C + 75% RH | 99.7 | 0.04 |
| 3 months 50°C | 100.1 | 0.05 |

### EXAMPLE 2

This example describes another preferred formulation of the association of enalapril and nitrendipine and the manufacturing method thereof.

| **Quantitative composition** | |
|---|---|
| ENALAPRIL MALEATE | 20.00 mg |
| MICRONIZED NITRENDIPINE | 5.00 mg |
| SODIUM BICARBONATE | 10.00 mg |
| CORN STARCH | 40.00 mg |
| SODIUM LAURYL-SULPHATE | 7.50 mg |
| MONOHYDRATED LACTOSE | 116.75 mg |
| POLYVINYLPYRROLIDONE | 11.20 mg |
| MICROCRYSTALLINE CELLULOSE | 80.00 mg |
| MAGNESIUM STEARATE | 2.20 mg |

### Manufacturing Method:

a) - Dissolve in a sufficient quantity of demineralized water the enalapril maleate and the corresponding quantity of sodium bicarbonate.
b)- Mix the nitrendipine suitably with 90 % of the corn starch, the sodium lauryl-sulphate, the monohydrate lactose, the polyvinylpyrrolidone and 20% of the monocrystalline cellulose, having previously sieved them.
c).- Using high-speed granulation equipment, granulate the products homogenized in section 2 with the solution obtained in section 1.
d).- Dry the granulated mass in fluidized bed equipment until a residual humidity of less than 1.5% is obtained.
e).- Calibrate the dry granulate. Add the remaining 10% of corn starch and the magnesium stearate, previous sieved, and homogenize with the calibrated granulate.
f).- Compress in conventional press.

Tables 3 and 4 below describe the stability results of the two active ingredients, enalapril and nitrendipine, in the formulation of this example, in function of time

**Table 3.-**

| Stability results of the enalapril of the formulation of example 2 in function of time. | | | |
|---|---|---|---|
| | | Degradation products | |
| | Content in % of Enalapril | Dicetopiperazin | Enalaprylic acid |
| Initial amb. T. | 102.1 | 0.00 | 0.04 |
| 3 months amb. T. | 101.7 | 0.00 | 0.08 |
| 3 months 40°C | 100.9 | 0.06 | 0.15 |
| 3 months 40°C + 75% RH | 101.2 | 0.08 | 0.17 |
| 3 months 50°C | 100.4 | 0.26 | 0.41 |

**Table 4.-**

| Stability results of the nitrendipine of the formulation of example 2 in function of time. | | |
|---|---|---|
| | Content in % of Nitrendipine | Degradation product Pyridinic derivative |
| Initial amb. T. | 99.1 | 0.00 |
| 3 months amb.T. | 98.1 | 0.01 |
| 3 months 40°C | 99.0 | 0.01 |
| 3 months 40°C + 75% RH | 98.8 | 0.02 |
| 3 months 50°C | 97.2 | 0.06 |

### EXAMPLE 3

In vitro dissolution trials have been carried out on 6 tablets from each of the formulations described in examples 1 and 2. The average results obtained for the enalapril and nitrendipine are described in the tables below.

**Table 5:**

| Results of dissolution of the enalapril of the formulations of examples 1 and 2 in function of time. | | | | |
|---|---|---|---|---|
| | EXAMPLE 1 | | EXAMPLE 2 | |
| Time (minutes) | % Enalapril dissolved | C.V.% | % Enalapril dissolved | C.V.% |
| 0.0 | 0.00 | 0.00 | 0.00 | 0.00 |
| 5.0 | 24.96 | 8.79 | 26.26 | 4.31 |
| 10.0 | 48.18 | 6.48 | 51.71 | 4.57 |
| 15.0 | 70.46 | 6.27 | 76.71 | 5.56 |
| 30.0 | 104.55 | 0.42 | 101.00 | 1.27 |
| 60.0 | 103.76 | 0.52 | 101.58 | 0.72 |

**Table 6:**

| Results of dissolution of the nitrendipine of the formulations of examples 1 and 2 in function of time. | | | | |
|---|---|---|---|---|
| | EXAMPLE 1 | | EXAMPLE 2 | |
| Time (minutes) | % Nitredipine dissolved | C.V.% | % Nitredipine dissolved | C.V.% |
| 0.0 | 0.00 | 0.00 | 0.00 | 0.00 |
| 5.0 | 23.10 | 9.82 | 25.95 | 5.57 |
| 10.0 | 45.35 | 7.47 | 49.55 | 4.41 |
| 15.0 | 66.98 | 6.53 | 71.70 | 4.04 |
| 30.0 | 97.90 | 0.36 | 93.51 | 1.07 |
| 60.0 | 98.76 | 0.47 | 95.18 | 0.01 |

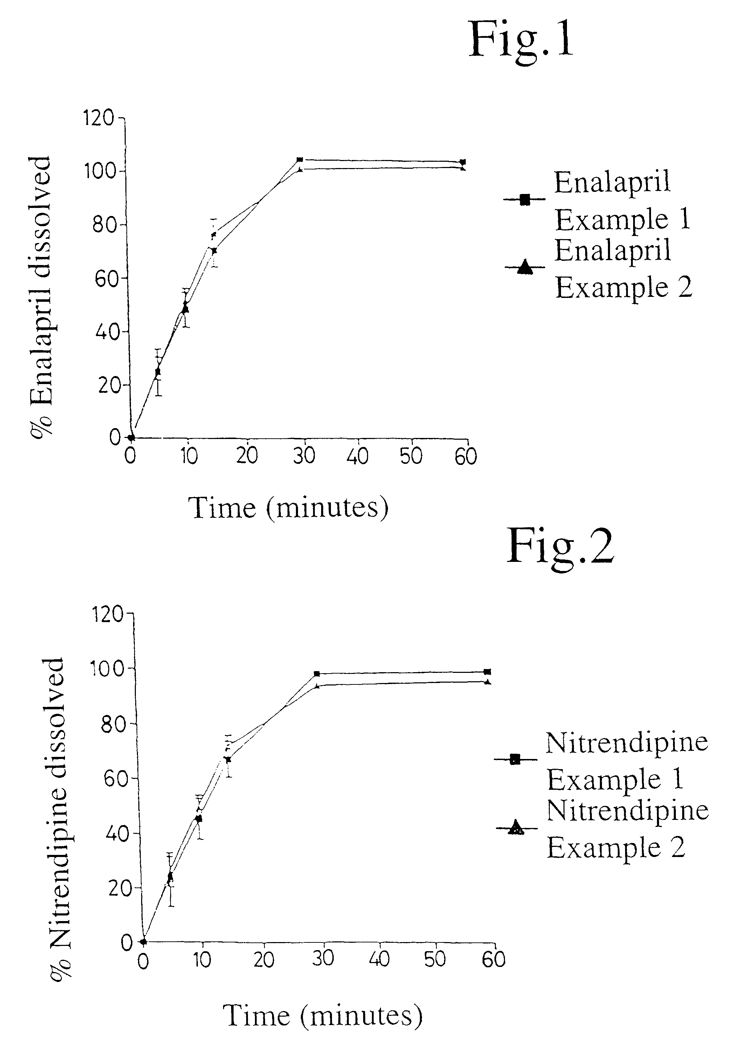

The above results are shown in figures 1 and 2, showing the accumulative profiles of in vitro dissolution of the enalapril and the nitrendipine for the 6 tablets tested of the formulations of examples 1 and 2.

### REFERENCE EXAMPLE 4

The pharmacological effect of the association of enalapril and nitrendipine of the invention was evaluated in two experimental models:

### a) Antihypertensive activity in the rat experimentally hypertense by aortic coarctation

The antihypertensive activity of the association of enalapril and nitrendipine was tested in comparison with each one of the isolated components in an experimental model of hypertension by arterial coarction in the rat.

Measurement of the arterial pressure was carried out with the animal conscious. The doses tested were as follows:
- Enalapril maleate 1 mg/kg (p.o.)
- Nitrendipine 3 mg/kg (p.o.)
- Enalapril maleate and Nitrendipine (1 and 3) mg/kg (p.o.)
- Enalapril maleate and Nitrendipine (0.5 and 1.5) mg/kg (p.o.)

The control group received the carrier (CMC Carboxymethylcellulose 1% en distilled water), the administration volume being 10 mg/kg (p.o.).

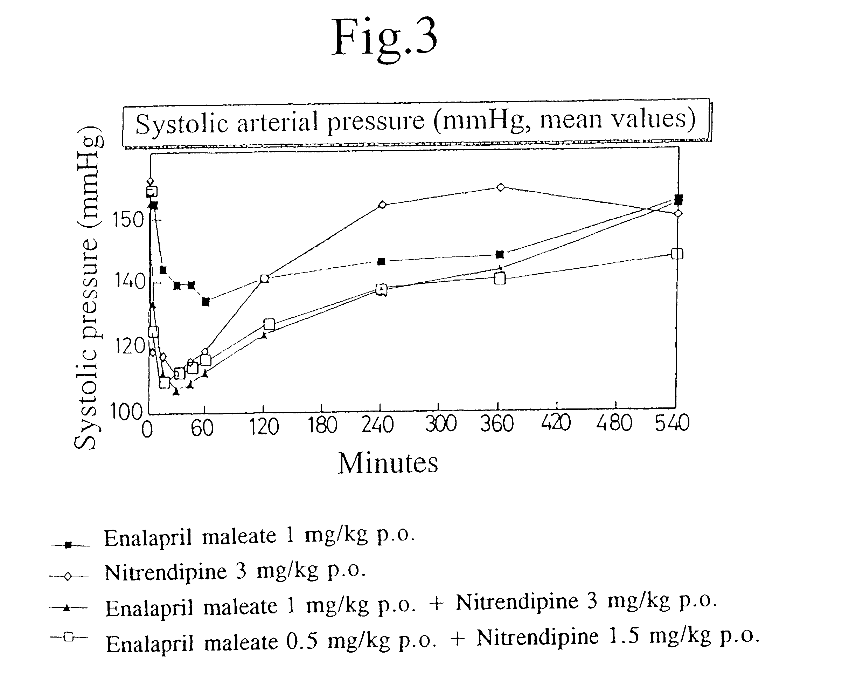

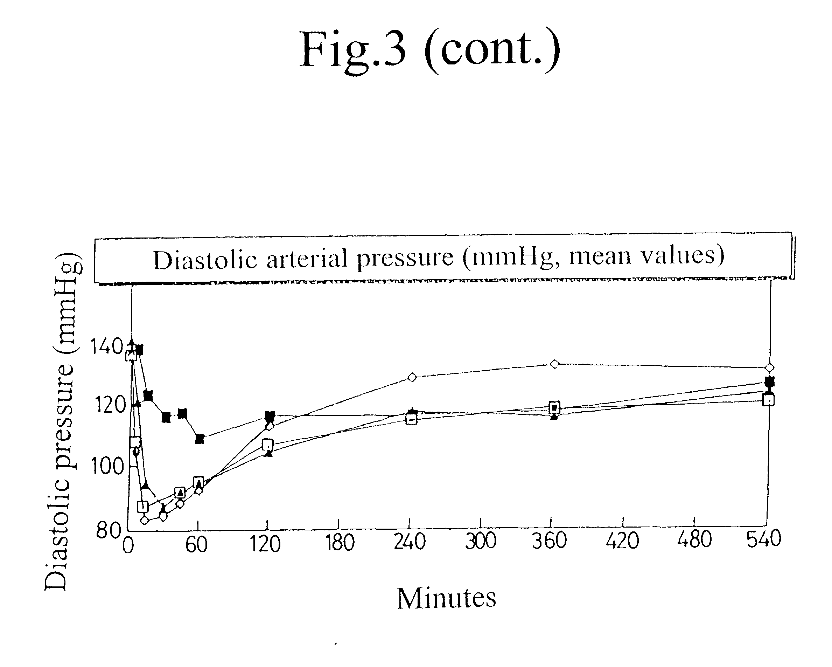

The data obtained are shown in table 7 and figure 3. Oral administration of enalapril maleate (1 mg/kg) revealed a moderate antihypertensive effect, but sustained for up to 6 hours following administration.

Oral administration of nitrendipine (3 mg/kg) produced a very marked antihypertensive effect, though not a very sustained one, with a return to basal values 4 hours following administration.

Combined administration of enalapril maleate (1 mg/kg, p.o.) and nitrendipine (3 mg/kg, p.o.) produced an antihypertensive effect greater than that of enalapril administered alone (1 mg/kg, p.o.). Trial at dosages lower than those initially used in the association (0.5 and 1.5 mg/kg, p.o.) of enalapril maleate and nitrendipine revealed an antihypertensive effect equivalent to the association (1 and 3 mg/kg, p.o.) of enalapril maleate and nitrendipine, together with a prolongation of the duration of the antihypertensive effect obtained with the individual treatments, from which it can be concluded that the association has a synergic effect.

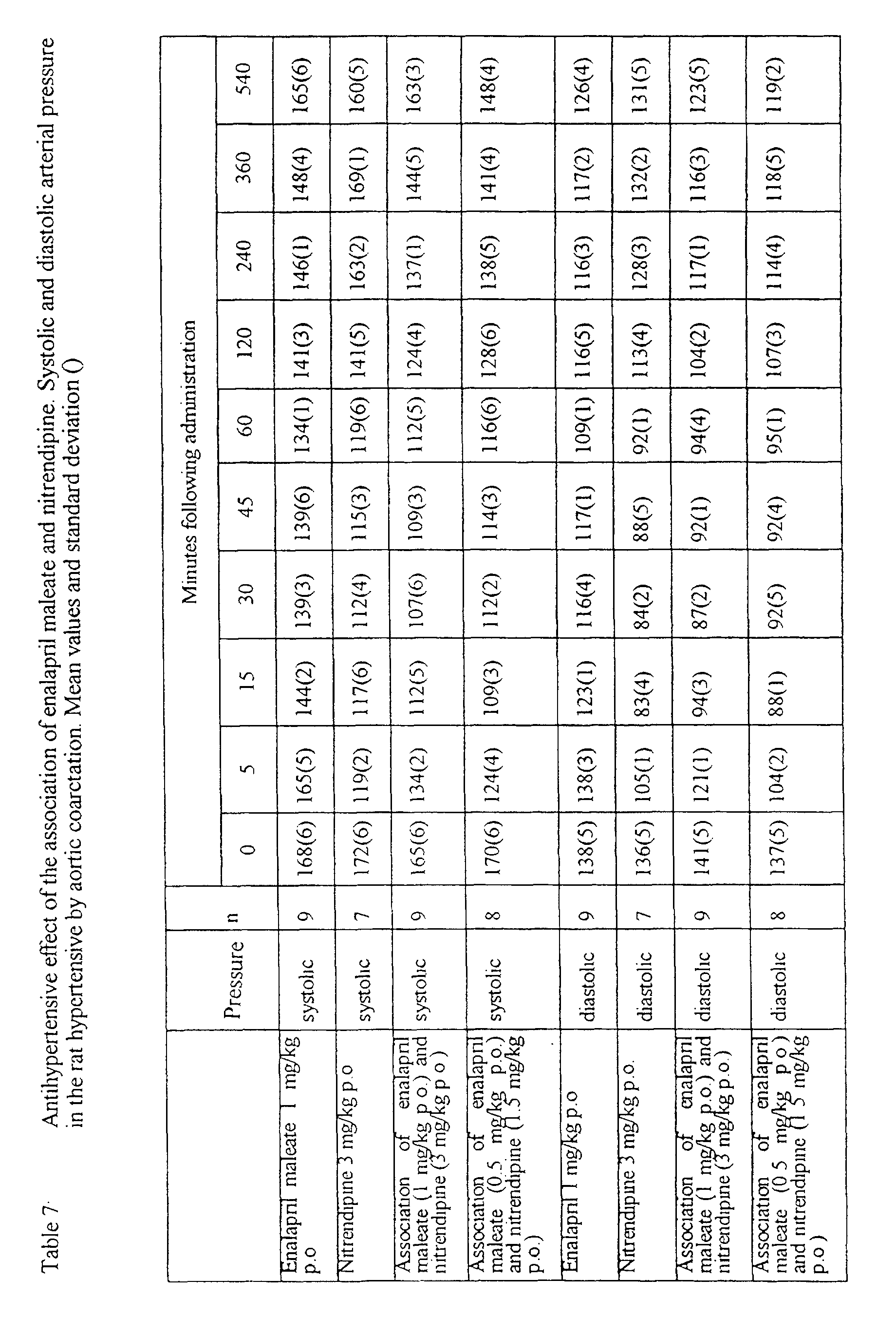

### b) Hypotensive activity in beagle dog with normal arterial tension

A study was made of the hypotensive effect of the association of enalapril and nitrendipine (1:1) in beagle 5 dog, with physiological data obtained from a 26-week study of chronic toxicity; enalapril maleate and nitrendipine (1:1) were administered at doses of 1 and 1; 3 and 3; and 6 and 6 mg/kg/day administered orally for 26 weeks in the form of capsules.

During the treatment period the systolic and diastolic arterial pressure were recorded and the mean pressure calculated in basal conditions following 1, 4, 13 and 26 weeks of treatment. At each of these periods the arterial pressure was recorded before administration and 4 5 and 8 hours after administration. The results obtained are shown in table 8.

Oral administration of enalapril maleate and nitrendipine (1:1) for 26 weeks at the doses of 1 and 1; 3 and 3; and 6 and 6 mg/kg/day revealed an appreciable 0 hypotensive effect right from the 1st week of treatment. The maximum effect appeared 4 hours after administration and was sustained for a further 8 hours following administration.

This hypotensive effect intensified over the time of treatment, with the lowest arterial pressure recorded at 13 5 weeks.

This study thus shows a clear pharmacological effect of the association of enalapril maleate and nitrendipine (1:1) at the doses of 1 and 1; 3 and 3; and 6 and 6 mg/kg/day (p.o.)

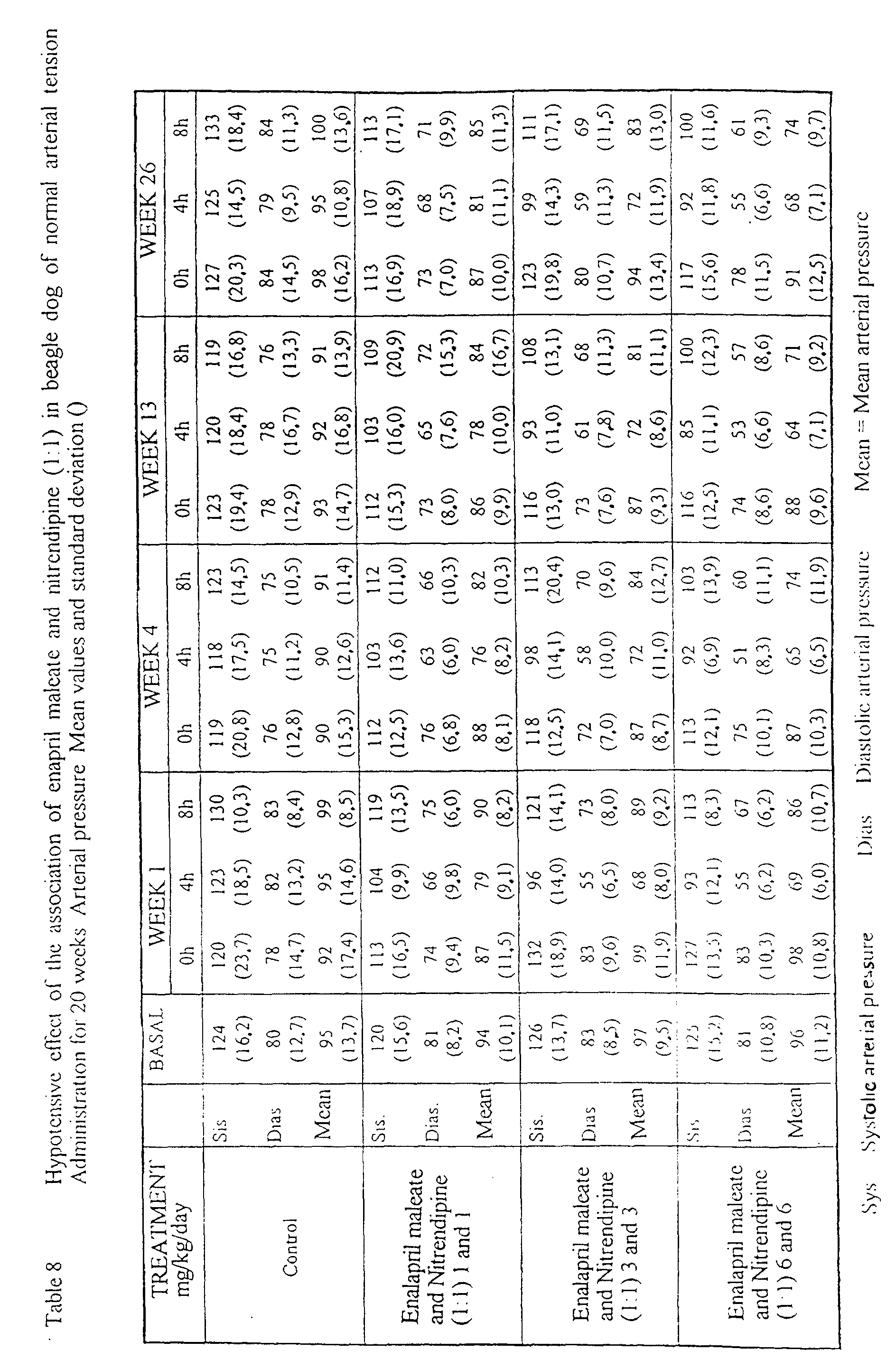

### REFERENCE EXAMPLE 5

A study was made of the pharmacokinetic interaction following a single dose of 20 mg of enalapril, 20 mg of nitrendipine and the fixed-dose association of 20 mg each of enalapril and nitrendipine as active ingredients, in 24 healthy volunteers (10 men and 14 women). For this purpose a randomized, open and crossed clinical trial was designed, using a Latin square of 3x3, with an off-period of 15 days between each of the treatment periods. In each treatment period 16 samples of blood were taken from each volunteer, from the basal moment up to +96 hours following administration, and the plasmatic concentrations of enalaprilate and nitrendipine. These date were used as the basis for calculation of the pharmacokinetic parameters which measure bioavailability in magnitude (AUC_{0-infinite}) and in speed (Cₘₐₓ , Cₘₐₓ/AUC_{0-infinite} and Tₘₐₓ₎) and a statistical analysis was made (ANOVA) to evaluate the statistical significance of the difference between the means of each of these parameters. This analysis revealed no statistically significant difference between the isolated ingestion of nitrendipine or enalapril and the combined ingestion of the fixed-dose association. The relative bioavailability (AUC_{0-infinite}) of enalaprilate following combined ingestion with respect to isolated ingestion was 1.12. The relative bioavailability (AUC_{0-infinite}) of nitrendipine following combined ingestion with respect to isolated ingestion was 0.91. It can therefore be concluded that there exists no clinically important pharmacokinetic interaction between nitrendipine and enalapril following administration of a single dose of 20 mg of each of the active ingredients separately and in their fixed-dose association in the pharmaceutical formulations of the invention.

### EXAMPLE 6

A placebo-controlled, open clinical trial was carried out in male patients with slight-moderate AHT, defined as systolic blood pressure values (SAT) between 165 and 144 mmHg and diastolic blood pressure values (DAT) between 95 and 105 mmHg, taken in decubitus position after 5 minutes' rest. Following a two-week placebo period, the patients complying with the slight-moderate AHT criteria received treatment with 5 mg nitrendipine once a day (9 patients) or 5 mg enalapril once a day (11 patients) for 2 weeks, at the end of which the patients who did not respond to the treatment (DAT < 90 mmHg) received combined treatment with the fixed-dose association of 5 mg nitrendipine and 5mg enalapril 5 mg, once a day, for a period of a further 2 weeks of treatment. The single-drug treatment only normalized blood pressure in 3 patients (15%), 2 treated with enalapril and 1 treated with nitrendipine. The combined treatment with the association normalized the blood pressure in 14 patients (82%). The mean reduction of the SAT and DAT figures with respect to the end of the placebo period was 5 mmHg/2 mmHg following the period of single-drug treatment (insignificant differences) and 24 mmHg/16 mmHg following the period of combined treatment with the fixed-dose association (p < 0.001). It can therefore be concluded that the combined administration of nitrendipine and enalapril in a fixed-dose association achieves effective control of slight-moderate AHT in most patients with doses which, when administered in single-drug therapy, have no clinically important effect in the treatment of AHT.

## Claims

1. Fixed-dose association of an angiotensin-converting enzyme inhibitor and of a calcium channel antagonist, **characterized in that** said association comprises a dose of (a) enalapril in the form of sodium salt and another dose of (b) nitrendipine micronized , the dose of enalapril being from 2.5 to 20 mg and the dose of nitrendipine being from 5 to 20 mg, and **in that** it is to be administered in single-dose galenic form.

2. Fixed-dose association as claimed in Claim 1, **characterized in that** the dose of enalapril ranges between 10 and 20 mg.

3. Fixed-dose association as claimed in Claim 1, **characterized in that** the dose of nitrendipine ranges between 5 and 10 mg.

4. Fixed-dose association as claimed in any of Claims 1 to 3, **characterized in that** the form of single dose is a capsule.

5. Fixed-dose association as claimed in any of Claims 1 to 3, **characterized in that** the form of single dose is a tablet.

6. Fixed-dose association as claimed in any of Claims 1 to 3, **characterized in that** the form of single dose is in single-dose sachets of powder for extemporaneous solution.

7. Pharmaceutically acceptable composition which comprises a fixed-dose association as claimed in any of Claims 1 to 6, **characterized in that** the gallenic form further includes a plastic diluting agent, preferably microcrystalline cellulose, a fragmentary diluting agent, preferably lactose, a disintegrating agent, preferable corn starch, an agglutinating agent, preferably polyvinylpyrrolidone, a wetting agent, preferably sodium lauryl-sulphate, and a lubricating agent, preferably magnesium stearate.

8. Method for obtaining a pharmaceutically acceptable composition as claimed in Claim 7, **characterized in that** it comprises:
(**a**) - dissolving enalapril maleate in water with an inorganic salt, selected from sodium bicarbonate;
(**b**)- mixing the micronized nitrendipine with a fraction of the disintegrating excipients, preferably corn starch, the wetting agent, preferably sodium lauryl-sulphate, the fragmentary diluting agent, preferably monohydrated lactose, the agglutinating agent, preferably polyvinylpyrrolidone and the plastic diluting agent, preferably microcrystalline cellulose, previously sieved;
(**c**)- granulating the products homogenized in section (b) with the solution obtained in section (a) ;
(**d**)- drying the granulated mass to a residual humidity of less than 3%, preferably less than 1.5%;
(**e**) - incorporating the lubricating agent, preferably magnesium stearate, and the remaining fraction of the disintegrating excipients and homogenizing the calibrated granulate;
(**f**)- compressing the granulate, or filling the capsules or the single-dose sachets.

9. Method as claimed in Claim 8, **characterized in that** the wetting agent and the agglutinating agent of stage (b) are previously dissolved in stage (a).

10. Utilization of a fixed-dose association of a dose of enalapril 1 and another dose of nitrendipine micronized for the manufacture of a medicament for the treatment of illnesses of the cardiovascular system, in particular arterial hypertension in mammals, especially in man.

## Patentansprüche

1. Festdosis-Assoziation aus einem Angiotensin-umwandelnden Enzyminhibitor und einem Calciumkanal-Antagonisten,
**dadurch gekennzeichnet, daß**
die Assoziation eine Dosis von (a) Enalapril in der Form des Natriumsalzes aufweist sowie eine andere Dosis von (b) mikronisiertem Nitrendipin, wobei die Dosis von Enalapril von 2,5 bis 20 mg beträgt, und die Dosis von Nitrendipin von 5 bis 20 mg reicht, und daß sie in einer galenischen Einzeldosis-Form verabreicht wird.

2. Festdosis-Assoziation nach Anspruch 1,
**dadurch gekennzeichnet, daß**
die Dosis von Enalapril von 10 bis 20 mg reicht.

3. Festdosis-Assoziation nach Anspruch 1,
**dadurch gekennzeichnet, daß**
die Dosis von Nitrendipin von 5 bis 10 mg reicht.

4. Festdosis-Assoziation nach einem der Ansprüche 1 bis 3,
**dadurch gekennzeichnet, daß**
die Einzeldosis-Form eine Kapsel ist.

5. Assoziation mit festgelegter Dosierung nach einem der Ansprüche 1 bis 3,
**dadurch gekennzeichnet, daß**
die Einzeldosis-Form eine Tablette ist.

6. Festdosis-Assoziationnach einem der Ansprüche 1 bis 3,
**dadurch gekennzeichnet, daß**
die Einzeldosis-Form in Einzeldosis-Pulvertütchen zur sofortigen Lösung vorliegt.

7. Pharmazeutisch akzeptable Zusammensetzung, umfassend eine Festdosis-Assoziation nach einem der Ansprüche 1 bis 6,
**dadurch gekennzeichnet, daß**
die galenische Form weiterhin ein plastisches Verdünnungsmittel aufweist, vorzugsweise mikrokristalline Cellulose, ein fragmentierendes Verdünnungsmittel, vorzugsweise Lactose, ein Zerfallsmittel, vorzugsweise Maisstärke, ein Agglutinationsmittel, vorzugsweise Polyvinylpyrrolidon, ein Netzmittel, vorzugsweise Natriumlaurylsulfat und ein Gleitmittel, vorzugsweise Magnesiumstearat.

8. Verfahren zur Herstellung einer pharmazeutischen akzeptablen Zusammensetzung nach Anspruch 7,
**dadurch gekennzeichnet, daß**
es die Schritte aufweist:
(a) Auflösen von Enalapril-Maleat in Wasser mit einem anorganischem Salz, ausgewählt aus Natriumbicarbonat;
(b) Mischen des mikronisiertem Nitrendipins mit einem Teil der Zerfalls-Exzipienten, vorzugsweise Maisstärke, dem Netzmittel, vorzugsweise Natriumlaurylsulfat, dem fragmentierenden Verdünnungsmittel, vorzugsweise monohydratisierter Lactose, dem Agglutinationsmittel, vorzugsweise Polyvinylpyrrolidon und dem plastischen Verdünnungsmittel, vorzugsweise mikrokristalliner Cellose, die zuvor gesiebt wurden;
(c) Granulieren der in Schritt (b) homogenisierten Produkte mit der in Schritt (a) erhaltenen Lösung;
(d) Trocknen der granulierten Masse bis auf eine Restfeuchtigkeit von weniger als 3 %, vorzugsweise weniger als 1,5 %;
(e) Einbeziehen des Gleitmittels, vorzugsweise Magnesiumstearat, und des verbleibenden Teils der Zerfalls-Exzipienten, und Homogenisieren des kalibrierten Granulats;
(f) Komprimieren des Granulats oder Befüllen der Kapsel oder der Einzeldosis-Tütchen.

9. Verfahren nach Anspruch 8,
**dadurch gekennzeichnet, daß**
das Netzmittel und das Agglutinationsmittel der Stufe (b) zuvor in Stufe (a) aufgelöst werden.

10. Verwendung einer Festdosis-Assoziation aus einer Enalapril-Dosis in der Form eines Natriumsalzes und einer anderen Dosis von mikronisiertem Nitrendipin zur Herstellung eines Medikaments zur Behandlung von Krankheiten des Herz-Kreislauf-Systems, insbesondere arterieller Hypertonie in Säugern, insbesondere in Menschen.

## Revendications

1. Association à dose fixe d'un inhibiteur de l'enzyme de conversion de l'angiotensine et d'un antagoniste de canal calcique, **caractérisée en ce que** ladite association comprend une dose de (a) enalapril sous la forme de sel de sodium, et d'une autre dose de (b) nitrendipine micronisée, la dose d'enalapril étant de 2,5 à 20 mg et la dose de nitrendipine étant de 5 à 20 mg, et **en ce qu'**elle doit être administrée sous une forme galénique à posologie unique.

2. Association à dose fixe selon la revendication 1, **caractérisée en ce que** la dose d'enalapril est située entre 10 et 20 mg.

3. Association à dose fixe selon la revendication 1, **caractérisée en ce que** la dose de nitrendipine est située entre 5 et 10 mg.

4. Association à dose fixe selon l'une quelconque des revendications 1 à 3, **caractérisée en ce que** la forme de posologie unique est une capsule.

5. Association à dose fixe selon l'une quelconque des revendications 1 à 3, **caractérisée en ce que** la forme de posologie unique est un comprimé.

6. Association à dose fixe selon l'une quelconque des revendications 1 à 3, **caractérisée en ce que** la forme de posologie unique comprend des sachets de poudre de dose unique pour une solution extemporanée.

7. Composition pharmaceutiquement acceptable qui comprend une association à dose fixe selon l'une quelconque des revendications 1 à 6, **caractérisée en ce que** la forme galénique comprend en outre un agent de dilution plastique, de préférence la cellulose microcristalline, un agent de dilution fragmentaire, de préférence le lactose, un agent de désintégration, de préférence l'amidon de maïs, un agent d'agglutination, de préférence la polyvinylpyrrolidone, un agent mouillant, de préférence le sodium lauryl sulfate, et un agent lubrifiant, de préférence le stéarate de magnésium.

8. Procédé pour obtenir une composition pharmaceutiquement acceptable selon la revendication 7, **caractérisé en ce qu'**il comprend
(a) - la dissolution du maléate d'enalapril dans de l'eau avec un sel inorganique, choisi parmi le bicarbonate de sodium ;
(b) - le mélange de la nitrendipine micronisée avec une fraction des excipients de désintégration, de préférence l'amidon de maïs, l'agent mouillant, de préférence le sodium lauryl sulfate, l'agent de dilution fragmentaire, de préférence le lactose monohydrate, l'agent d'agglutination, de préférence la polyvinylpyrrolidone, et l'agent de dilution plastique, de préférence la cellulose microcristalline, précédemment tamisée ;
(c) - la granulation des produits homogénéisés dans la section (b) avec la solution obtenue dans la section (a) ;
(d) - le séchage de la masse granulée à une humidité résiduelle inférieure à 3%, de préférence inférieure à 1,5% ;
(e) - l'incorporation de l'agent lubrifiant, de préférence le stéarate de magnésium, et la fraction restante des excipients de désintégration, et l'homogénéisation du granulé calibré ;
(f) - la compression du granulé, ou le remplissage des capsules ou des sachets de dose unique.

9. Procédé selon la revendication 8, **caractérisé en ce que** l'agent mouillant et l'agent d'agglutination du stade (b) sont précédemment dissous au stade (a).

10. Utilisation d'une association à dose fixe d'une dose d'enalapril sous la forme de sel de sodium, et d'une autre dose de nitrendipine micronisée, pour la fabrication d'un médicament pour le traitement des maladies du système cardiovasculaire, en particulier (hypertension artérielle chez les mammifères, en particulier chez l'homme.
